(19) Europäisches Patentamt
European Patent Office
Office européen des brevets

(11) Veröffentlichungsnummer : **0 183 970**
**B1**

(12) # EUROPÄISCHE PATENTSCHRIFT

(45) Veröffentlichungstag der Patentschrift : **09.09.87**

(21) Anmeldenummer : **85113571.5**

(22) Anmeldetag : **25.10.85**

(51) Int. Cl.⁴ : **C 07 C 43/196**, C 07 C 69/14, C 07 C 69/24, C 11 B 9/00, A 23 L 1/226

(54) **p-Alkoxy-cyclohexyl-alkanole und -alkanolester, deren Herstellung und Verwendung als Riechstoffe.**

(30) Priorität : **08.11.84 DE 3440825**

(43) Veröffentlichungstag der Anmeldung : **11.06.86 Patentblatt 86/24**

(45) Bekanntmachung des Hinweises auf die Patenterteilung : **09.09.87 Patentblatt 87/37**

(84) Benannte Vertragsstaaten : **BE CH DE FR GB IT LI NL**

(56) Entgegenhaltungen :
**DE-C- 2 804 075**
**FR-A- 1 294 932**
**JOURNAL OF THE CHEMICAL SOCIETY, CHEMICAL COMMUNICATIONS, 1980, Seiten 762-763, Royal Society of Chemistry; T. KAMETANI et al.: "Studies on the stereochemical course of selenium-assisted cyclisation: biogenetic-type synthesis in the p-menthan series"**

(73) Patentinhaber : **BASF Aktiengesellschaft**
**Carl-Bosch-Strasse 38**
**D-6700 Ludwigshafen (DE)**

(72) Erfinder : **Degner, Dieter, Dr.**
**Kurpfalzstrasse 8**
**D-6701 Dannstadt-Schauernheim (DE)**
Erfinder : **Gramlich, Walter, Dr.**
**Auf der Hoehe 11**
**D-6803 Edingen-Neckarhausen (DE)**
Erfinder : **Hoffmann, Werner, Dr.**
**Fritzsche Dodge & Olcott Inc. 76 Ninth Avenue**
**New York, N.Y. 10113-0009 (US)**
Erfinder : **Schuster, Ludwig, Dr.**
**Weinheimer Strasse 44**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 183 970 B1

Jouve, 18, rue St-Denis, 75001 Paris, France

**Beschreibung**

Die Erfindung betrifft p-Alkoxy-cyclohexyl-alkanole und deren Ester der allgemeinen Formel I

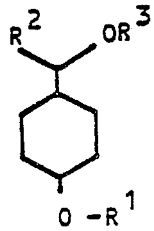

(I)

in der

$R^1$ für einen $C_1$- bis $C_7$-Alkylrest, insbesondere einen Methyl- oder tert.-Butylrest,

$R^2$ für eine $C_1$- bis $C_3$-Alkylgruppe, insbesondere eine Methyl- oder Ethylgruppe und

$R^3$ für Wasserstoff, einen Acetyl-, Propionyl- oder Butyrylrest steht,

sowie Riechstoffkompositionen enthaltend diese Verbindungen I und Verfahren zu deren Herstellung. Die neuen Verbindungen stellen eine Klasse neuer Holzriechstoffe dar. Ähnlich weit wie der Begriff, Holzriechtstoff, weisen auch die neuen Verbindungen verschiedene Geruchstypen auf, die vom erdigkrautigen Typ bis zum feinen und teuren Sandelholztyp reichen. Insbesondere Verbindungen, in denen $R^1$ für einen tert.-Butylrest steht, weisen feine Sandelholzduftnoten auf. Sie können als Riechstoffe sowie als Bestandteil von Riechstoffkompositionen verwendet werden.

Das ostindische Sandelholzöl gehört zu den bekannten Naturstoffen, die über extrem wertvolle Riechstoffeigenschaften verfügen.

Natürliches Sandelholzöl wird durch Dampfdestillation aus Kernholz und Wurzeln von Sandelholz erhalten, das in Indien und Malaysia vorkommt. Es ist ein wichtiger Bestandteil von Parfümmischungen mit orientalischer bzw. exotischer Note.

Sandelholz ist eine spezielle Pflanze, die auf den Wurzeln anderer Bäume lebt, wobei lange Schößlinge von seinen Wurzeln ausgehen. Es wird aus Samen kultiviert und es sind 25 bis 30 Jahre erforderlich, bis Sandelholzöl aus ihm gewonnen werden kann.

Entsprechend sind die Quellen in Asien spärlicher geworden und die Versorgung mit diesem wertvollen Öl ist in den letzten Jahren knapp geworden, was zu einer Preiserhöhung des Sandelholzöles geführt hat.

Die Hauptbestandteile des natürlichen Sandelholzöles sind alpha-Santalol und beta-Santalol.

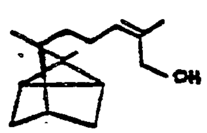

α-Santalol

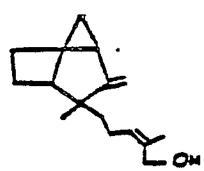

ß-Santalol

Da trotz zahlreicher Versuche keine kostengünstigen Synthesen für alpha- und beta-Santalol gefunden wurden, wurden schon zahlreiche Versuche unternommen, das natürliche Sandelholzöl durch synthetische Verbindungen mit Sandelholzduft zu ersetzen.

So werden beispielsweise in dem Dragoco-Report, Heft 11/12 (1981), S. 39 Verbindungen mit recht unterschiedlichen Strukturen genannt, die einen sandelholzartigen Geruch aufweisen. Allen diesen Verbindungen gemeinsam ist, daß es sich um relativ kompliziert aufgebaute Verbindungen handelt, die nur auf recht aufwendige Weise zugänglich sind.

Es war daher die Aufgabe der Erfindung, neue Verbindungen zu finden, die einerseits technisch auf einfache und billige Weise hergestellt werden können und trotzdem einen feinen aber starken Duft nach Holz, insbesondere nach Sandelholz aufweisen, so daß man durch sie das natürliche Sandelholzöl auch in anspruchsvollen Riechstoffkompositionen ersetzen kann.

Es wurde nun gefunden, daß die p-Alkoxy-cyclohexyl-alkanole und deren Ester der allgemeinen Formel I, interessante Holznoten und speziell das 1-(p-tert.-Butoxy-cyclohexyl)-1-ethanol, insbesondere dessen cis-Isomeres, die begehrte Duftnote des ostindischen Sandelholzöls aufweisen.

Es war zwar schon aus loc. cit. bekannt, daß 1-(α-Hydroxy-ethyl)-4-(3-methyl-pent-3-yl)-cyclohexan der Formel III

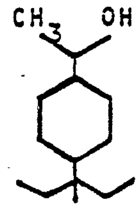

(III)

einen sandelholzartigen Geruch aufweist, doch wird dort auch ausgeführt, daß bereits bei geringfügiger Modifikation dieses Moleküls erhebliche Abschwächungen der Geruchseigenschaften auftreten.

Es war daher überraschend, daß die aus technisch gut zugänglichen und billigen Vorprodukten, wie Anisaldehyd oder tert.-Butoxy-benzaldehyd, in technisch einfach durchführbaren Schritten und in guten Ausbeuten erhältlichen p-Alkoxy-cyclohexyl-alkanole der Formel I einen Sandelholzduft aufweisen, der insbesondere im Falle des 1-(p-Butoxy-cyclohexyl)-ethanols dem des ostindischen Sandelholzöls ähnlicher ist, als der anderer synthetischer Produkte.

Die neuen Duftstoffe lassen sich auf verschiedenen einfachen Wegen in an sich bekannten und technisch leicht realisierbaren Schritten herstellen. So erhält man beispielsweise 1-(p-Methoxy-cyclohexyl)-1-propanol durch Umsetzen von Anisol mit Propionsäureanhydrid in Gegenwart von $ZnCl_2$ und anschließendes Hydrieren des erhaltenen p-Methoxy-propiophenons in sehr guten Ausbeuten. 1-(p-tert.-Butoxy-cyclohexyl)-1-ethanol erhält man durch Grignardmethylierung von 4-tert.-Butoxy-benzaldehyd und anschließende Hydrierung, wobei der 4-tert.-Butoxy-benzaldehyd seinerseits neuerdings beispielsweise durch elektrochemische Oxidation von tert.-Butoxy-toluol (s. Patentanmeldung P 33 22 399.8) großtechnisch erhältlich ist.

Gegenstand der Erfindung ist daher auch ein Verfahren zur Herstellung von p-Alkoxy-cyclohexyl-alkanolen und deren Ester der allgemeinen Formel I

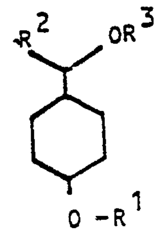

(I)

gemäß Anspruch 1, das dadurch gekennzeichnet ist, daß man eine Verbindung der allgemeinen Formeln IIa oder IIb

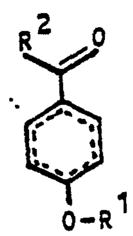

(IIa)

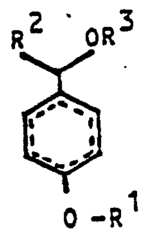

(IIb)

in der $R^1$, $R^2$ und $R^3$ die in Anspruch 1 angegebenen Bedeutungen haben und jeweils 3 oder 2 nichtbenachbarte, eine oder keine der gestrichelten Linien eine zusätzliche Bindung bedeuten, in an sich bekannter Weise hydriert, oder aber eine Verbindung der allgemeinen Formel IIc

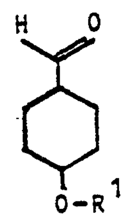

(IIc)

in an sich bekannter Weise mit einer den Rest $R^2$ abgebenden metallorganischen Verbindung umsetzt, und anschließend die erhaltenen Alkanole gewünschtenfalls verestert.

Als Ausgangsverbindungen können demnach Verbindungen der allgemeinen Formeln IIa oder IIb verwendet werden, die keine, eine, zwei oder drei nichtbenachbarte Doppelbindungen enthalten. Bei der Auswahl der geeigneten Vorprodukte wird man sich von Wirtschaftlichkeitsgesichtspunkten leiten lassen. Sie lassen sich auf verschiedenen Wegen herstellen bzw. sind als Zwischenprodukte in vielen Fällen kommerziell zu erwerben. Das folgende Schema gibt eine knappe, verständlicherweise unvollständige Übersicht über Herstellungsmöglichkeiten der erfindungsgemäßen Verbindungen I wieder :

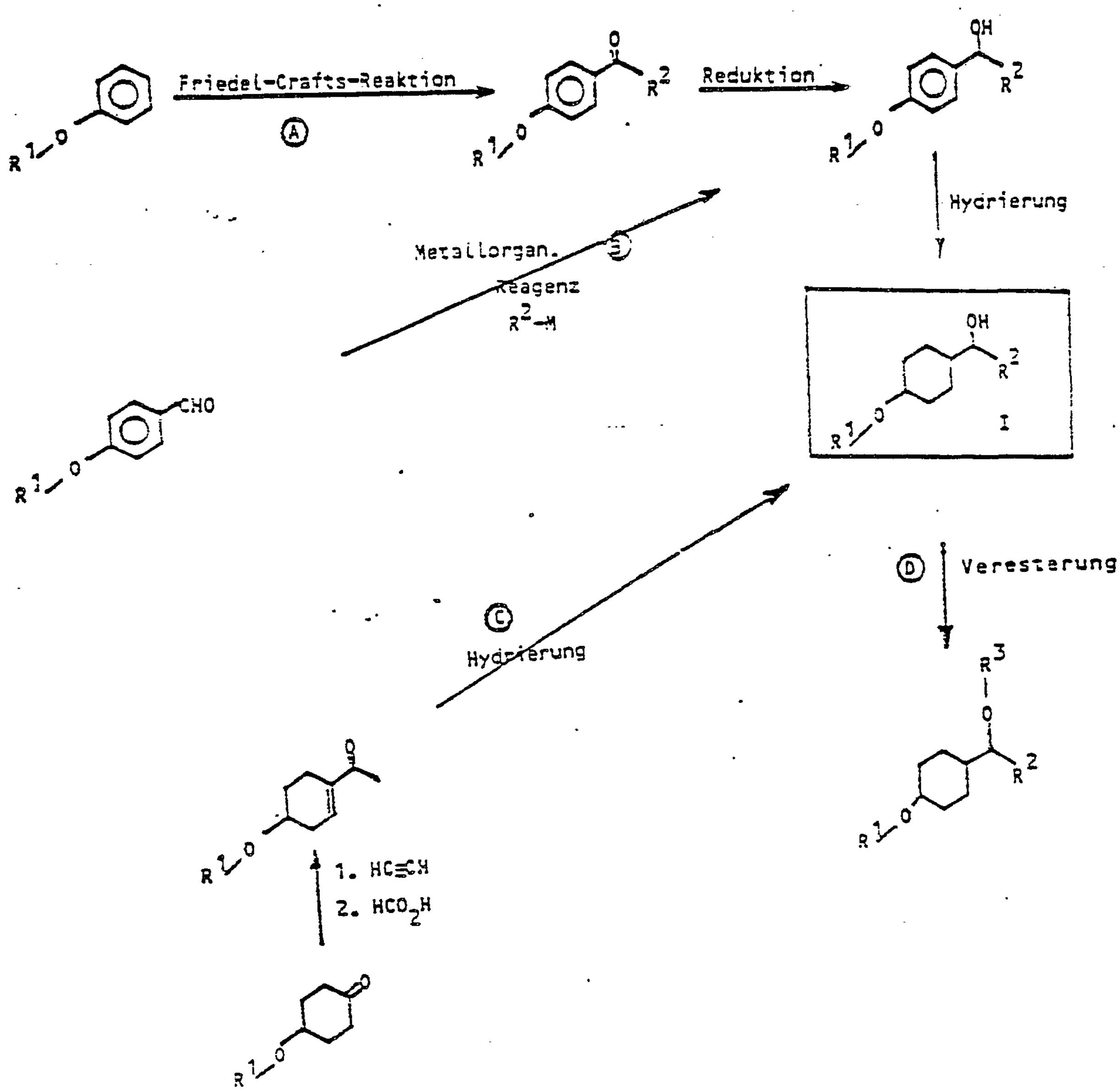


So lassen sich nach Reaktionsweg Ⓐ aromatische Alkyl- ether unter Friedel-Crafts-Bedingungen z. B. mit Carbonsäureanhydriden oder Carbonsäurechloriden in Gegenwart eines Lewissäure-Katalysators zu den entsprechenden acylierten Aromaten umsetzen, welche sich dann in ein oder zwei Stufen zu den Verbindungen I reduzieren lassen.

Beim zweistufigen Verfahren kann man im ersten Schritt bekannte Reagenzien wie $NaBH_4$, $LiAlH_4$ oder eine katalytische Reduktion z. B. mit Raney-Nickel einsetzen. Den nachfolgenden Schritt führt man vorzugsweise unter Verwendung von Rhodium- oder Ruthenium-Katalysatoren durch. Diese Katalysatoren hydrieren das aromatische System ohne gleichzeitige Spaltung der Etherfunktionen.

Diese Ruthenium- bzw. Rhodium-Katalysatoren sind auch geeignet, die alkoxilierten Aromaten in einem Schritt zu den Verbindungen I zu hydrieren.

Als Katalysatoren werden meist 0,5 bis 10 Gew.% Ru- bzw. Rh-Katalysatoren auf Aluminiumoxid- oder Kohle-Träger verwendet. Es lassen sich jedoch auch die reinen Metalle einsetzen.

Die Bedingungen bezüglich Katalysator, Lösungsmittel, Reaktionstemperatur, Wasserstoffdruck und Reaktionszeit können in weiten Grenzen variiert werden. Die Hydrierung wird beim einstufigen Verfahren bevorzugt zwischen 25 °C und 200 °C durchgeführt.

Als Lösungsmittel für die Hydrierung können beispielsweise Alkanole, wie Methanol oder Ethanol ; Ether, wie Tetrahydrofuran ; Kohlenwasserstoffe, wie Pentan ; und Säuren wie Essigsäure eingesetzt werden ; die Hydrierung kann jedoch auch ohne Lösungsmittel durchgeführt werden.

Beim Syntheseweg Ⓑ wird der entsprechende aromatische Aldehyd mit geeigneten metallorganischen Reagenzien $R^2$-M, wie Alkylgrignard-Verbindungen, umgesetzt. Bevorzugt wird dabei der Aldehyd zu einer Lösung eines Metallalkyls in einem inerten Lösungsmittel zugetropft und danach das entstandene Produkt, z. B. mit einer Säure, hydrolysiert.

Unter den metallorganischen Verbindungen werden bevorzugt Grignard-Reagenzien, wie

Alkylmagnesiumchlorid, -bromid oder -jodid oder Lithiumalkyle wie Methyllithium verwendet.

Beim Syntheseweg © werden alkoxylierte Cyclohexenylmethylketone, die man u. a. durch eine Ethinylierungs/Umlagerungs-Reaktionsfolge erhalten kann, auf einem ein- oder zweistufigen Wege zu Verbindungen vom Typ I hydriert. Beim wirtschaftlicheren Einstufen-Hydrierverfahren werden meist Palladium, Raney-Nickel, Cobalt, Platin, Ruthenium oder Rhodium als Hydrierkatalysatoren eingesetzt. Die Reaktionsbedingungen für dieses Einstufenverfahren sind nicht kritisch und können in weiten Grenzen bezüglich Lösungsmittel, Reaktionstemperatur, Wasserstoffdruck und Katalysatormenge variiert werden. Bevorzugter Katalysator ist Raney-Nickel, die Menge bezüglich Keton liegt meist zwischen 5 und 20 Gew.%. Der Temperaturbereich liegt im allgemeinen zwischen 100 und 150 °C, der Druckbereich zwischen 5 und 100 bar Wasserstoffdruck. Die erfindungsgemäßen Alkoxycyclohexyl-alkanole der Formel I, lassen sich mit Carbonsäureanhydriden oder Carbonsäurehalogeniden in an sich bekannter Weise in die entsprechenden Ester der Formel I überführen (Syntheseweg D), die ihrerseits wieder andere interessante Holznoten aufweisen. So erhält man beispielsweise durch Umsetzung von 1-(p-Methoxy-cyclohexyl)-1-propanol mit Essigsäureanhydrid das entsprechende Acetat, welches eine interessante pilzige Waldnote aufweist.

Interessant ist noch zu erwähnen, daß beispielsweise das bei der Hydrierung von 1-(p-tert.-Butoxy-phenyl)-1-ethanol als cis/trans-Gemisch anfallende 1-(p-tert.-Butoxy-cyclohexyl)-1-ethanol durch Fraktionierung über eine 50-Boden-Kolonne in das cis- und das trans-Isomere aufgespalten werden kann, und beide Isomere eine unterschiedliche Duftnote aufweisen. Während das cis-Produkt eine feine Sandelholznote aufweist, besitzt das trans-Isomere eine Note, die mit der des westindischen Cedernholzöles vergleichbar ist.

Die neuen Verbindungen der Erfindung können als Riechstoffe und somit als Bestandteile von Riechstoffkompositionen wie Parfums and Parfumbasen sowie zur Parfümierung kosmetischer und technischer Produkte (Seifen, Waschmittel u. a.) verwendet werden.

Von ganz besonderer Bedeutung unter den neuen Verbindungen sind 1-(p-tert.-Butoxy-cyclohexyl)-1-ethanol und 1-[4-(2-Methyl-2-butyloxy)-cyclohexyl]-1-ethanol, da sie beide, insbesondere die erstgenannte Verbindung einen so feinen und dennoch intensiven Sandelholzduft aufweisen, daß sie als Substitute für natürliches Sandelholzöl verwendet werden können. Gegenüber dem natürlichen Sandelholzöl weisen sie den Vorteil auf, viel stabiler als dieses zu sein. Darüber hinaus sind sie gut und daher erheblich kostengünstiger und fast unbegrenzt herstellbar. 1-(p-Methoxy-cyclohexyl)-1-propanol riecht zwar etwas schwächer sandelholzartig, zeigt jedoch immer noch eine recht interessante feine Holznote.

Die Strukturen der neuen Verbindungen wurden durch zutreffende IR-, $^1$H-NMR- und Massenspektren sowie Elementaranalysen gesichert.

## Beispiel 1

a) Herstellung von p-Methoxy-propiophenon

Ein Gemisch aus 108 g (1 mol) Anisol, 130 g (1 mol) Propionsäureanhydrid und 7 g $ZnCl_2$ (wasserfrei) wurde 5 Stunden (h) unter Rückfluß zum Sieden erhitzt und danach die entstehende Propionsäure überdestilliert. Nachdem 50 g Destillat angefallen waren, wurde etwas abgekühlt und der Rückstand bei 10 mbar fraktioniert. Man erhielt 103 g p-Methoxy-propiophenon (Kp. 150 °C/10 mbar, Kp. 273 °C/1 013 mbar).

b) Herstellung von 1-(p-Methoxy-cyclohexyl)-1-propanol

Ein Gemisch aus 100 g p-Methoxy-propiophenon, 250 ml Methanol und 5 g eines 5 %igen Ruthenium/Aktivkohle-Katalysators wurde bei einer Temperatur von 120 °C und einem Druck von 150 bar hydriert, bis die Wasserstoffaufnahme praktisch beendet war. Nach dem Entfernen des Katalysators wurde dann das Lösungsmittel bei 1 013 mbar abdestilliert und der Rückstand fraktioniert. Man erhielt 92 g einer farblosen Flüssigkeit ($Kp._{0,01}$ = 73-75 °C) die eine feine holzige Note aufwies.

## Beispiel 2

Herstellung von 1-(p-Methoxy-cyclohexyl)-1-propyl-acetat

Ein Gemisch aus 161 g (0,93 mol) 1-(p-Methoxy-cyclohexyl)-1-propanol und 306 g (3 mol) Essigsäureanhydrid wurden auf 120 bis 140 °C erhitzt und innerhalb von 3 h ein Gemisch von 260 g Acetanhydrid/Essigsäure abdestilliert. Nach Abkühlen wurde das Reaktionsgemisch mit 150 ml Diethylether versetzt, mit Natriumcarbonat neutralisiert und das Lösungsmittel abgedampft. Der erhaltene Rückstand wurde danach fraktioniert, wobei man 189 g einer Fraktion erhielt (Kp. 78 °C/0.1 mbar), die eindeutig als 1-(p-Methoxy-cyclohexyl)-1-propylacetat identifiziert werden konnte. Die Verbindung wies einen interessanten pilzartigen Waldgeruch auf.

Beispiel 3

a) Herstellung von 1-(p-tert.-Butoxy-phenyl)-1-ethanol

Zu 2 l einer 1,7 molaren Methylmagnesiumchlorid-Lösung (3,4 mol) in Tetrahydrofuran wurden bei 25 bis 30 °C 356 g (2 mol) 4-tert.-Butoxybenzaldehyd zugetropft. Nach Zulaufende wurde noch 1,5 h bei 25 °C nachgerührt und dann vorsichtig mit 100 ml $H_2O$ hydrolysiert. Danach wurden noch 1,3 l Wasser und schließlich 0,4 l einer 37 %igen Schwefelsäure zugegeben. Nach 1 h Nachrührzeit ließ man absitzen, trennte die beiden Phasen, extrahierte die untere wäßrige Phase noch einmal mit 400 ml Diethylether und vereinigte die organischen Phasen. Anschließend wurde noch einmal mit 100 ml wäßr. Natriumhydrogencarbonat-Lösung und dann mit 100 ml gesättigter Kochsalzlösung gewaschen.

Nach Entfernen des Lösungsmittels wurden 380 g einer Fraktion erhalten (Kp. 96-99 °C/0,02 mbar), die nach allen analytischen und spektroskopischen Daten als 1-(p-tert.-Butoxy-phenyl)-1-ethanol identifiziert werden konnte (Ausbeute 97,9 %).

b) Herstellung von 1-(p-tert.-Butoxy-cyclohexyl)-1-ethanol

In einem Autoklaven wurden 200 g 1-(p-tert.-Butoxy-phenyl)-1-ethanol in 500 g Dioxan in Gegenwart von 1 g Rutheniumhydroxid mehrfach mit Stickstoff und Wasserstoff gespült und dann bei einer Temperatur von 140 °C und einem Wasserstoffdruck von 300 bar bis zur Druckkonstanz hydriert.

Nach Entfernen des Katalysators wurde zunächst das Lösungsmittel entfernt und danach der Rückstand bei 0,01 mbar destilliert. Man erhielt 181 g eines farblosen Öls, das bei 61 °C/0,01 mbar siedete, und das nach spektroskopischen und analytischen Daten ein cis/trans Gemisch (80 : 20) von 1-(p-tert.-Butoxy-cyclohexyl)-1-ethanol darstellt. Die Ausbeute betrug 88 % der Theorie.

Die Substanz hat eine feine Note nach ostindischem Sandelholzöl.

Ähnliche Ergebnisse erzielt man, wenn man die gemäß Beispiel Ia erhaltene, gewaschene organische Phase direkt hydriert.

c) Trennung von cis/trans-1-(p-tert.-Butoxy-cyclohexyl)-1-ethanol

Ein bei der Hydrierung von 1-(p-tert.-Butoxy-phenyl)-1-ethanol als 75 : 25 cis/trans-Gemisch angefallenes 1-(p-tert.-Butoxy-(cyclohexyl)-1-ethanol wurde durch Fraktionierung über eine 50-Böden-Kolonne in die Einzelkomponenten getrennt.

Destillationsdaten :

1) cis-1-(p-tert.-Butoxy-cyclohexyl)-1-ethanol : Kp. 72 °C/0.01 mbar
2) trans-1-(p-tert.-Butoxy-cyclohexyl)-1-ethanol : Kp. 78 °C/0.01 mbar

Während das cis-Produkt eine feine Sandelholznote aufweist, besitzt das trans-Isomere eine Note, die mit der des westindischen Cedernholzöles vergleichbar ist.

Beispiel 4

Herstellung von 1-(p-tert.-Butoxy-cyclohexyl)-1-ethylacetat

Ein Gemisch aus 200 g (1 mol) 1-(p-tert.-Butoxy-cyclohexyl)-1-ethanol und 306 g (3 mol) Essigsäureanhydrid wurde 30 Min. unter Rückfluß zum Sieden erhitzt. Innerhalb von 3 h wurden nun bei Normaldruck 260 g eines Gemisches aus Essigsäure und überschüssigem Acetanhydrid abdestilliert und das noch verbleibende restliche Acetanhydrid anschließend unter vermindertem Druck (20 mbar) entfernt. Der auf 20 °C abgekühlte Rückstand wurde mit Wasser, wäßriger Bicarbonat-Lösung und erneut mit Wasser gewaschen und danach fraktioniert. Man erhielt 218 g einer Fraktion, die bei 0.1 mbar/90 °C überdestilliert und eine interessante krautige fruchtige Note aufweist.

Anwendungsbeispiel

Repräsentatives Beispiel für ein Parfüm

| | Gew.-Teile |
|---|---|
| 1-(p-tert.-Butoxy-cyclohexyl)-1-ethanol | 350 |
| Vertofix® (Fixativ von International Flavors and Fragrances) | 100 |
| p-tert.-Butyl-cyclohexylacetat | 150 |
| γ-Methyljonon | 100 |
| Linalylacetat | 80 |
| Linalool | 40 |

| | |
|---|---|
| Benzylacetat | 50 |
| Cedrol | 10 |
| Dimethylbenzylcarbinylacetat | 15 |
| alpha-Hexyl-zimtaldehyd | 20 |
| 2-Heptyl-cyclopentanon | 10 |
| Undecanal (20 % in Dipropylenglykol) | 2 |
| Phenylethylalkohol | 20 |
| Limonen | 23 |
| Hydroxycitronellal | 30 |
| | 1 000 |

Der erzielte Geruchseffekt der Komposition mit der erfindungsgemäßen Verbindung entspricht ziemlich genau demjenigen, der durch Zugabe der gleichen Menge an ostindischem Sandelholzöl anstelle von 350 Gew.-Teilen 1-(p-tert.-Butoxy-cyclohexyl)-1-ethanol zu der sonst gleichen Riechstoffkomposition erzielt wird.

**Patentansprüche**

1. p-Alkoxy-cyclohexyl-alkanole und deren Ester der allgemeinen Formel I

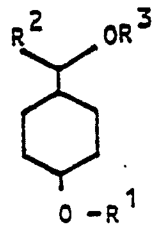

(I)

in der
$R^1$ für einen $C_1$- bis $C_7$-Alkylrest,
$R^2$ für eine $C_1$- bis $C_3$-Alkylgruppe und
$R^3$ für Wasserstoff, einen Acetyl-, Propionyl oder Butyrylrest steht.

2. 1-(p-tert.-Butoxy-cyclohexyl)-1-ethanol.

3. Verfahren zur Herstellung von p-Alkoxy-cyclohexyl-alkanolen und deren Estern der allgemeinen Formel I

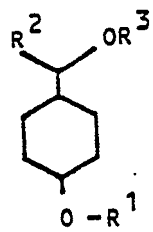

(I)

gemäß Anspruch 1, dadurch gekennzeichnet, daß man eine Verbindung der allgemeinen Formeln IIa oder IIb

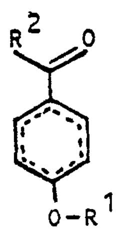

(IIa)

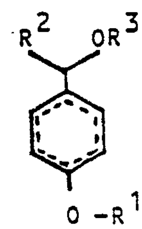

(IIb)

in der $R^1$, $R^2$ und $R^3$ die in Anspruch 1 angegebene Bedeutung haben und jeweils 3 oder 2 nichtbenachbarte, eine oder keine der gestrichelten Linien eine zusätzliche Bindung bedeuten, in an sich bekannter Weise hydriert, oder aber eine Verbindung der allgemeinen Formel IIc

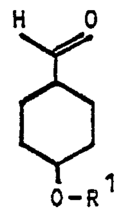

, (IIc)

in an sich bekannter Weise mit einer den Rest $R^2$ abgebenden metallorganischen Verbindung umsetzt, und anschließend die erhaltenen Alkanole gewünschtenfalls verestert.

4. Verfahren zur Verbesserung des Geruchs von Riechstoffkompositionen, dadurch gekennzeichnet, daß man solchen Kompositionen ein p-Alkoxycyclohexyl-alkanol oder dessen Ester der allgemeinen Formel I

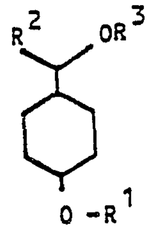

(I)

in der $R^1$, $R^2$ und $R^3$ die in Anspruch 1 angegebene Bedeutung haben, zusetzt.

5. Riechstoffkompositionen, enthaltend ein p-Alkoxy-cyclohexyl-alkanol oder einen seiner Ester der allgemeinen Formel I

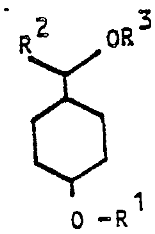

(I)

in der $R^1$, $R^2$ und $R^3$ die in Anspruch 1 angegebene Bedeutung haben.

6. Riechstoffe, enthaltend 1-(p-tert.-Butoxy-cyclohexyl)-1-ethanol.

**Claims**

1. A p-alkoxycyclohexylalkanol and its esters of the general formula I

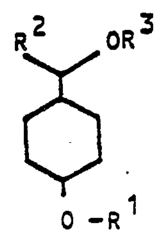

(I)

where
$R^1$ is $C_1$-$C_7$-alkyl,
$R^2$ is $C_1$-$C_3$-alkyl, and
$R^3$ is hydrogen, acetyl, propionyl or butyryl.

2. 1-(p-Tert.-butoxycyclohexyl)-ethan-1-ol.

3. A process for the preparation of a p-alkoxycyclohexylalkanol or its esters of the general formula I

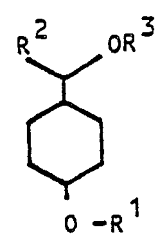

(I)

as claimed in claim 1, wherein a compound of the general formula IIa or IIb

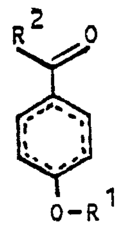

(IIa)

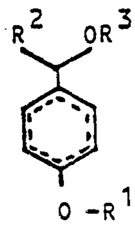

(IIb)

where $R^1$, $R^2$ and $R^3$ have the meanings given in claim 1, and 3 or 2 non-adjacent dashed lines are each an additional bond, or one or none of the dashed lines is an additional bond, is hydrogenated in a conventional manner, or a compound of the general formula IIc

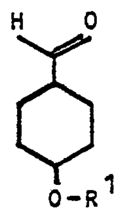

(IIc)

is reacted in a conventional manner with an organometallic compound which donates a radical $R^2$, and, if desired, the resulting alkanol is then esterified.

4. A process for improving the odour of perfumery compositions, wherein a p-alkoxycyclohexylalkanol or one of its esters of the general formula I

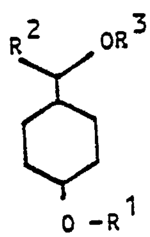

(I)

where $R^1$, $R^2$ and $R^3$ have the meanings stated in claim 1, is added to such a composition.

5. A perfumery composition containing a p-alkoxycyclohexylalkanol or one of its esters of the general formula I

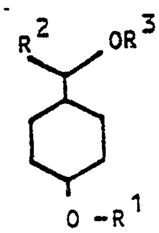

(I)

where $R^1$, $R^2$ and $R^3$ have the meanings given in claim 1.

6. A perfume containing 1-(p-tert.-butoxycyclohexyl)-ethan-1-ol.

**Revendications**

1. p-Alcoxy-cyclohexyl-alcanols et leurs esters de la formule générale I

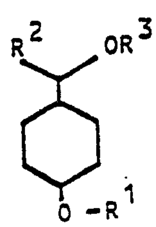

(I)

dans laquelle

$R^1$ désigne un radical alkyle en $C_1$ à $C_7$,

$R^2$ un radical alkyle en $C_1$ à $C_3$ et

$R^3$ un atome d'hydrogène ou un radical acétyle, propionyle ou butyryle.

2. Le 1-(p-tert.-butoxy-cyclohexyl)-1-éthanol.

3. Procédé de préparation de p-alcoxy-cyclohexyl-alcanols et de leurs esters de la formule générale I

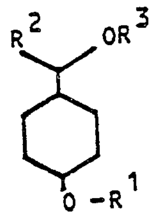

(I)

suivant la revendication 1, caractérisé en ce que, soit l'on soumet un composé de l'une des formules générales IIa et IIb

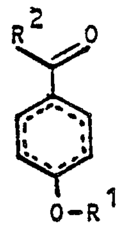

(IIa)

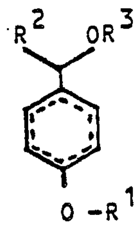

(IIb)

dans lesquelles $R^1$, $R^2$ et $R^3$ possèdent la signification définie dans la revendication 1, trois lignes ou deux lignes non adjacentes ou une ou aucune des lignes en trait interrompu pouvant représenter une liaison additionnelle, à une hydrogénation connue en soi, soit l'on fait réagir un composé de la formule générale IIc

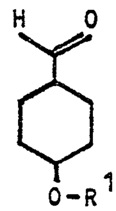

(IIc)

de façon connue en soi avec un composé organo-métallique capable de céder un reste $R^2$, les alcanols obtenus pouvant ensuite, si on le désire, être estérifiés.

4. Procédé pour améliorer le parfum de compositions de matières odorantes, caractérisé en ce que l'on incorpore à une telle composition un p-alcoxy-cyclohexyl-alcanol ou un de ses esters de la formule générale I

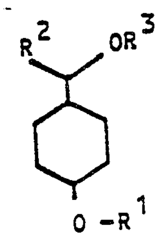

(I)

dans laquelle $R^1$, $R^2$ et $R^3$ possèdent la signification définie dans la revendication 1.

5. Compositions de matières odorantes, contenant un p-alcoxy-cyclohexyl-alcanol ou un de ses esters de la formule générale

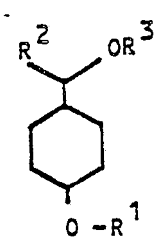

(I)

dans laquelle $R^1$, $R^2$ et $R^3$ possèdent la signification définie dans la revendication 1.

6. Composition odorante, contenant du 1-(p-tert.-butoxy-cyclohexyl)-1-éthanol.